# EUROPÄISCHE PATENTSCHRIFT

(11) **EP 2 271 317 B1**
(45) Veröffentlichungstag und Bekanntmachung des Hinweises auf die Patenterteilung: **19.04.2017**
(21) Anmeldenummer: 09726303.2
(22) Anmeldetag: 24.03.2009
(51) Int. Cl.: A61K 9/50, A61K 31/4184, A61K 31/4439, A61K 31/519, A61K 31/60

(54) **VERFAHREN ZUR HERSTELLUNG VON SÄUREPELLETS**
METHOD FOR MANUFACTURING ACID PELLETS
PROCÉDÉ DE PRODUCTION DE GRANULES D'ACIDE

(30) Priorität: 28.03.2008 EP 08153668
(43) Veröffentlichungstag der Anmeldung: 12.01.2011
(73) Patentinhaber: Boehringer Ingelheim International GmbH, 55216 Ingelheim am Rhein (DE)
(72) Erfinder: MAIER, Johann-Georg, 55216 Ingelheim am Rhein (DE)
(74) Vertreter: Simon, Elke Anna Maria
(86) Internationale Anmeldenummer: PCT/EP2009/053468
(87) Internationale Veröffentlichungsnummer: WO 2009/118321

(56) Entgegenhaltungen:
- EP-A- 0 257 344
- EP-A- 1 894 561
- WO-A-03/074056
- WO-A-2008/022932
- LUO Y ET AL: "Dry coating, a novel coating technology for solid pharmaceutical dosage forms", INTERNATIONAL JOURNAL OF PHARMACEUTICS, ELSEVIER BV, NL, vol. 358, no. 1-2, 27 March 2008 (2008-03-27), pages 16-22, XP022698703, ISSN: 0378-5173, DOI: 10.1016/J.IJPHARM.2008.03.028 [retrieved on 2008-03-27]

## Beschreibung

Die Erfindung betrifft ein verbessertes Verfahren zur Herstellung von annähernd sphärischen/kugelförmigen Weinsäurestarterpellets, die zur Herstellung von wirkstoffhaltigen Arzneimittelformulierungen geeignet sind, sowie die so erhältlichen Pellets als solche und deren Verwendung als Ausgangsmaterial zur Herstellung wirkstoffhaltigen Arzneimittelformulierungen.

### Hintergrund der Erfindung

Aus dem Stand der Technik sind Pellets, d.h. kleine runde Kügelchen als Grundlage für pharmazeutische Darreichungsformen bekannt. Häufig werden sogenannte "neutrale" Pellets bestehend aus Maisstärke und Saccharose als Grundlage für pharmazeutische Darreichungsformen in Pelletform verwendet. Ebenfalls möglich ist die Verwendung von Starterpellets, welche überwiegend aus einer organischen Säure bestehen. Eine solche Pelletformulierung ist beispielsweise in der WO 03/074056 offenbart. Bei diesen Formulierungen handelt es sich um Zusammensetzungen bei denen auf ein annähernd sphärisches Kernmaterial, das aus der pharmazeutisch akzeptablen organischen Säure besteht oder diese enthält, eine binde- und gegebenenfalls trennmittelhaltige Wirkstoffschicht, die das Kernmaterial umschließt, aufgetragen ist. Die Kernschicht und die Wirkstoffschicht sind dabei durch eine so genannte Isolierschicht voneinander getrennt. Der schematische Aufbau einer solchen Wirkstoffformulierung ist in Figur 1 der WO 03/074056 dargestellt. Zusätzlich beschreibt die WO 08/02293 ein System zur kontrollierten Wirkstoffabgabe, das auf einem annähernd sphärischen Kernmaterial aus einem oder mehreren pharmazeutisch akzeptablen pHbeeinflussenden Stoffen, umgeben von einer optionalen Isolierschicht und einer Schutzschicht aus einem oder mehreren pharmazeutisch akzeptablen wasserunlöslichen Polymeren aufbaut. Als pharmazeutisch akzeptable pH-beeinflussende Stoffe zur Herstellung des annähernd sphärisches Kernmaterial werden unter anderem Essigsäure, Weinsäure und Zitronensäure erwähnt.

Die Herstellung von solchen Säurestarterpellets mit der erwünschten annähernd kugelförmigen/sphärischen Geometrie erweist sich jedoch unerwartet als schwierig. Insbesondere kann es immer wieder zu starken Abweichungen von der gewünschten kugelförmigen Symmetrie, etwa zu Agglomeraten aus einer größeren Kugel mit außen anhaftenden kleineren Klümpchen, sogenannte Satelliten, kommen. Bei Pelletformulierungen, in denen der Starterpellet von einer säureempfindlichen Wirkstoffschicht umschlossen wird, können diese Satelliten nach Auftragen der Isolierschicht abbrechen und dadurch Fehlstellen in der Isolierung verursachen. Dies wiederum wirkt sich nachteilig auf die Lagerstabilität der Wirkstoffpellets aus.

Es ist Aufgabe der vorliegenden Erfindung so genannte Starterpellets bereitzustellen, die eine gleichmäßige, kugelähnliche Geometrie aufweisen. Darüber hinaus sollten sie nur geringfügig potentielle Fehlstellen in der Isolierung durch Satelliten aufweisen. Von besonderer Bedeutung ist die möglichst ideale Kugelform und eine geringe Oberflächenrauhigkeit für säureempfindliche Wirkstoffe, bei denen Fehlstellen der Isolierung durch abgebrochene Satelliten oder durch zu rauhe Oberfläche zu großer Weinsäurepulverpartikel zu deutlich verschlechterten Lagerstabilitäten und damit Haltbarkeiten des fertigen Produkts führen kann. Aus diesem Grund ist es bei säureempfindlichen Wirkstoffen auch erforderlich, die Isolierschicht als solche in hoher Reproduzierbarkeit und gleichbleibend guter Qualität aufzubringen.

### Detaillierte Beschreibung der Erfindung

Die vorliegende Erfindung betrifft ein Verfahren zur Herstellung von Starterpellets, welche die vorstehend genannte Aufgabe lösen.

Das erfindungsgemäße Verfahren ist durch eine Reihe von Teilschritten gekennzeichnet. Zunächst erfolgt die Herstellung des Kerns **1** aus pharmazeutisch akzeptabler organischer Säure. Im Rahmen der vorliegenden Erfindung gelangt zur Herstellung des Kerns **1** Weinsäure zur Anwendung. Das so erhaltene Kernmaterial **1** wird anschließend durch Aufsprühen einer Isoliersuspension **2** in so genannte isolierte Weinsäurekerne **3** überführt. Im Rahmen der vorliegenden Erfindung werden die isolierten Weinsäurekerne **3** gegebenenfalls auch als isolierte Weinsäurepellets **3** oder auch als Starterpellets bezeichnet.

Die Herstellung der Kerns **1** erfolgt aus Weinsäurepartikeln mit einer Partikelgröße im Bereich von 0,4-0,6 mm (bestimmt durch Luftstrahlsiebung) auf die eine Lösung aus Weinsäure und Bindemittel aufgesprüht wird.

Zur Herstellung der Lösung wird wie folgt vorgegangen. Weinsäure wird zunächst gemeinsam mit einem geeigneten Bindemittel, bevorzugt mit Acacia (Gummi Arabicum) bei erhöhter Temperatur, vorzugsweise bei einer Temperatur im Bereich von 30-70°C, besonders bevorzugt im Bereich von 40-60°C in Wasser gelöst. Pro eingesetztes Kilogramm Weinsäure werden bevorzugt 0,1-0,3 kg, besonders bevorzugt 0,15-0,25 kg, insbesondere etwa 0,2 kg Acacia verwendet. Die Menge an Wasser beträgt vorzugsweise 0,6-1,0 kg, bevorzugt 0,7-0,9 kg, insbesondere etwa 0,8 kg pro eingesetztes Kilogramm Weinsäure.

Erfindungsgemäß bevorzugt wird bei der vorstehend genannten Temperatur zunächst eine klare Lösung aus Acacia in Wasser hergestellt. Liegt diese vor, erfolgt anschließend die Zugabe der Weinsäure bei vorzugsweise konstanter Temperatur und unter fortgesetztem Rühren. Nach beendeter Zugabe wird wenigstens 1 Stunde, vorzugsweise zwischen 3 und 10, besonders bevorzugt 4 - 8, besonders bevorzugt 5 - 6 Stunden nachgerührt.

Die so erhaltene Lösung wird auf Weinsäurepartikel mit einer Partikelgröße von 0,4-0,6 mm aufgesprüht. Der Anteil der Partikel mit der vorstehend genannten Partikelgröße sollte wenigstens 90%, vorzugsweise wenigstens 95%, besonders bevorzugt wenigstens bei 97% liegen. Hierzu werden die Weinsäurepartikel in einem geeigneten Kessel vorgelegt. Bei dem Kessel handelt es sich vorzugsweise um einen Kessel, bei dem das Mischen und die Bewegung der Partikel durch die Drehung des Kessels erfolgt. Solche Kessel sind in unterschiedlichen Ausführungen im Stand der Technik bekannt und werden gegebenenfalls auch als Trommelcoater bezeichnet. Diesbezüglich sei beispielsweise auf die Offenbarungen der EP 80199, WO 83/03052, WO 95/19713 oder auch WO 06/134133 verwiesen. Im Rahmen der vorliegenden Erfindung werden im erfindungsgemäßen Verfahren einsetzbare Kessel gegebenenfalls auch als Horizontalkessel bezeichnet.

Auf die durch Rotation in Bewegung gehaltenen Partikel wird dann die nach vorstehend beschriebener Vorgehensweise hergestellte Säuregummilösung aufgesprüht.

Im Rahmen der vorliegenden Erfindung wird das vorgelegte Sprühgut gegebenenfalls auch als Pelletbett bezeichnet. Der Begriff Pellet ist im Rahmen der vorliegenden Erfindung als gleichbedeutend mit dem Begriff Partikel oder Kern anzusehen.

Pro vorgelegtes Kilogramm Weinsäurepartikel werden erfindungsgemäß bevorzugt 0,8 - 1,6 kg, besonders bevorzugt 1,0 - 1,4 kg, besonders bevorzugt 1,2 kg der vorstehend genannten Säuregummilösung aufgesprüht.

Die Zuluftmenge ist im erfindungsgemäßen Verfahren abhängig von der Chargengröße. Pro Kilogramm vorgelegte Weinsäurekerne liegt die normierte Zuluftmenge erfindungsgemäß bevorzugt in einem Bereich von 0,5 - 2 (m³/h)/kg, bevorzugt 0,75 - 1,5 (m³/h)/kg, besonders bevorzugt 0,9 - 1,1 (m³/h)/kg. Unter Zuluftmenge wird die Menge an Trockenluft verstanden, die pro Stunde in das rotierende Pelletbett eingebracht wird.

Werden in einem Ansatz beispielsweise 1000 kg Weinsäurekerne vorgelegt, entspricht eine normierte Zuluftmenge von 1,0 (m³/h)/kg einer faktischen Zuluftmenge von 1000 m³/h. Die Temperatur der zur Trocknung zugeführten Zuluft liegt erfindungsgemäß bevorzugt unterhalb von 90°C, besonders bevorzugt unterhalb von 80°C. Idealerweise sollte die Temperatur der Zuluft in einem Bereich von 35°-75°C liegen.

Die Pellettemperatur (die Temperatur des vorliegenden Pelletbetts) liegt erfindungsgemäß bevorzugt in einem Bereich von 30 - 50°C, besonders bevorzugt bei 36 - 44°C, idealerweise liegt sie bei 38 - 42°C.

Der Differenzdruck beträgt bevorzugt 1 - 3 mbar, besonders bevorzugt 1,5 - 2,5 mbar, besonders bevorzugt 1,8 - 2,2 mbar. Der Differenzdruck ist der Druckunterschied zwischen Kesseldruck und Umgebungsdruck. Der Kessel soll vorzugsweise Unterdruck haben damit kein Säurestaub austritt.

Das Aufsprühen erfolgt bei definierter Sprührate. Unter Sprührate wird die Menge an Säuregummilösung verstanden, die pro Stunde auf das rotierende Pelletbett aufgesprüht wird. Die Sprührate ist im erfindungsgemäßen Verfahren abhängig von der Chargengröße. Pro Kilogramm vorgelegte Weinsäurekristalle liegt die normierte Sprührate erfindungsgemäß bevorzugt in einem Bereich von 0,2 - 0,4 (kg/h)/kg, bevorzugt 0,25 - 0,35 (kg/h)/kg, besonders bevorzugt 0,28 - 0,32 (kg/h)/kg. Werden in einem Ansatz beispielsweise 1000 kg Weinsäurekristalle vorgelegt, entspricht eine normierte Sprührate von 0,3 (kg/h)/kg einer faktischen Sprührate von 300 kg/h.

Nach Aufsprühen eines ersten Teils der Säuregummilösung auf die Weinsäurepartikel der Partikelgröße 0,4-0,6 mm und Verteilen der Lösung durch Rotation des Kessels wird feines Weinsäurepulver auf die feuchten Weinsäurepartikel aufgepudert. Bei genanntem Weinsäurepulver handelt es sich um feine Weinsäurepartikel mit einer Partikelgröße von < 75, bevorzugt < 50 Mikrometern (bestimmt durch Luftstrahlsiebung). Der Anteil der Partikel mit der vorstehend genannten Partikelgröße sollte wenigstens bei 85%, bevorzugt wenigstens bei 90%, besonders bevorzugt bei wenigstens 94% liegen. Pro vorgelegtes Kilogramm Weinsäurepartikel werden erfindungsgemäß bevorzugt 0,4 - 1,2 kg, besonders bevorzugt 0,6 - 1,0 kg, besonders bevorzugt 0,8 kg des vorstehend genannten Weinsäurepulvers verwendet. Nach Aufpudern mit genanntem Weinsäurepulver wird das Sprühgut getrocknet, bis eine Guttemperatur von etwa 30-50°C, bevorzugt etwa 40°C erreicht ist. Hieran schließt sich erneut das Aufsprühen der Säuregummilösung an.

Um die gleichmäßige Bildung kugelförmiger Partikel zu gewährleisten erfolgt das Aufsprühen der Säuregummilösung und das Aufpudern mit Weinsäurepulver im Wechsel. Hierbei werden die Gesamtmengen der Säuregummilösung und des Weinsäurepuders in wenigstens 100, bevorzugt 150 bis 350, besonders bevorzugt 200 bis 300, besonders bevorzugt etwa 250 ähnlich großen Portionen zugeführt und die vorstehend beschriebenen Prozessschritte entsprechend oft wiederholt.

Nach beendetem Prozess werden die erhaltenen Kerne **1** getrocknet. Die Trocknung erfolgt bevorzugt bei einer Temperatur von 50-70°C, bevorzugt 55-65°C über einen Zeitraum von 24 - 72 Stunden, bevorzugt 36 - 60 Stunden.

Nach Herstellung der Weinsäurekerne **1** ist eine so genannte Isolierung des Kernmaterials erforderlich. Hierbei wird eine Isolierschicht um den Weinsäurekern aufgebracht, die eine Wechselwirkung von Wirkstoff mit Weinsäurekern im späteren Produkt verhindert.

Die Isolierung des Kernmaterials erfolgt durch Aufsprühen einer Isoliersuspension **2** auf die gemäß vorstehend beschriebener Vorgehensweise erhaltenen Weinsäurekerne **1**. Zur Herstellung der Isoliersuspension **2** wird Ethanol im Ansatzbehälter vorgelegt und unter Rühren Hydroxypropylmethylcellulose und Dimethylpolysiloxan eingetragen und gelöst, danach wird Talkum zugegeben und suspendiert.

Die Verwendung von Hydroxypropylmethylcellulose und Talkum hat sich gegenüber der Verwendung von beispielsweise Gummi Arabicum und Talkum als überlegen herausgestellt. Durch die Verwendung von Hydroxypropylmethylcellulose neben Talkum ist es möglich in reproduzierbarer Art und Weise eine Isolierschicht gleichbleibender Qualität herzustellen. Diese Qualität und Reproduzierbarkeit wurde im großtechnischen Maßstab erprobt.
Zur Darstellung der Isoliersuspension **2** werden pro Kilogramm Ethanol bevorzugt 0,04 - 0,06 kg, besonders bevorzugt 0,046 - 0,05 kg Hydroxypropylmethylcellulose verwendet. Neben der Verwendung von Hydroxypropylmethylcellulose hat es sich als erfindungsgemäß besonders bevorzugt erwiesen, der Isoliersuspension **2** Dimethylpolysiloxan zu zusetzen, um Schaumbildung zu vermeiden. Die Menge an Dimethylpolysiloxan, die zur Herstellung der Isoliersuspension **2** unter Rühren zugesetzt wird, beträgt pro Kilogramm Ethanol bevorzugt 0,6 - 1,2 g, besonders bevorzugt 0,8 - 0,9 g. Schließlich wird Talkum unter Rühren zugesetzt und suspendiert. Pro Kilogramm Ethanol gelangen bevorzugt 0,04 - 0,06 kg, besonders bevorzugt 0,046 - 0,05 kg Talkum zum Einsatz.

Ein Aspekt der vorliegenden Erfindung betrifft eine ethanolische Isoliersuspension **2**, die Hydroxypropylmethylcellulose, vorzugsweise in den vorstehend genannten Mengen enthält. Ein weiterer Aspekt der vorliegenden Erfindung betrifft eine ethanolische Isoliersuspension **2**, die neben Hydroxypropylmethylcellulose Dimethylpolysiloxan, vorzugsweise in den vorstehend genannten Mengen, enthält. Ein weiterer Aspekt der vorliegenden Erfindung betrifft eine ethanolische Isoliersuspension **2**, die neben Hydroxypropylmethylcellulose und Dimethylpolysiloxan ferner Talkum, vorzugsweise in den vorstehend genannten Mengen, enthält. Ein weiterer Aspekt der vorliegenden Erfindung betrifft eine ethanolische Isoliersuspension **2**, die gemäß der vorstehend beschriebenen Vorgehensweise erhältlich ist.

Ein weiterer Aspekt der vorliegenden Erfindung betrifft die Verwendung der ethanolischen Isoliersuspension **2**, zur Isolierung von Weinsäurekernen **1**. Ein weiterer Aspekt der vorliegenden Erfindung betrifft die Verwendung der ethanolischen Isoliersuspension **2**, als Ausgangsmaterial zur Herstellung einer Arzneimittelformulierung des Dabigatranetexilatmethansulfonats.

Die so hergestellte Isoliersuspension **2** wird in einem kontinuierlichen Sprühprozess in einem konventionellen Horizontalcoater auf die zuvor hergestellten Weinsäurepellets **1** aufgesprüht. Pro Kilogramm vorgelegter Weinsäurekerne **1** werden 0,5 - 0,8 kg, bevorzugt 0,55 - 0,75 kg, besonders bevorzugt 0,6 - 0,7 kg Isoliersuspension aufgesprüht.

Das Aufsprühen erfolgt bei definierter Sprührate. Unter Sprührate wird die Menge an Isoliersuspension **2** verstanden, die pro Stunde auf die Pellets **1** aufgesprüht wird. Die Sprührate ist im erfindungsgemäßen Verfahren abhängig von der Chargengröße. Pro Kilogramm vorgelegte Weinsäurepellets **1** liegt die normierte Sprührate erfindungsgemäß bevorzugt in einem Bereich von 0,01 - 0,1 (kg/h)/kg, bevorzugt 0,02 - 0,04 (kg/h)/kg, besonders bevorzugt 0,025 - 0,035 (kg/h)/kg. Werden in einem Ansatz beispielsweise 1200 kg Weinsäurekerne vorgelegt, entspricht eine normierte Sprührate von 0,027 (kg/h)/kg einer faktischen Sprührate von 32 kg/h. Werden in einem Ansatz beispielsweise 600 kg Weinsäurekerne vorgelegt, entspricht eine normierte Sprührate von 0,035 (kg/h)/kg einer faktischen Sprührate von 21 kg/h.

Während dieses kontinuierlichen Prozesses werden die Kerne kontinuierlich mit einer Zuluft von bis zu 70°C, vorzugsweise von 25 - 70°C getrocknet.

Unter Zuluftmenge wird die Menge an Trockenluft verstanden, die pro Stunde in das rotierende Pelletbett eingebracht wird. Die Zuluftmenge ist im erfindungsgemäßen Verfahren abhängig von der Chargengröße. Pro Kilogramm ursprünglich vorgelegte Weinsäurekerne **2** liegt die normierte Zuluftmenge erfindungsgemäß bevorzugt in einem Bereich von 1,0 - 2,5 (m³/h)/kg. bevorzugt 1,2 - 2,0 (m³/h)/kg, besonders bevorzugt 1,40 - 1,85 (m³/h)/kg.
Werden in einem Ansatz beispielsweise 600 kg Weinsäurekerne **2** vorgelegt, entspricht eine normierte Zuluftmenge von 1,83 (m³/h)/kg einer faktischen Zuluftmenge von 1100 m³/h. Werden in einem Ansatz beispielsweise 1200 kg Weinsäurekerne **3** vorgelegt, entspricht eine normierte Zuluftmenge von 1,42 (m³/h)/kg einer faktischen Zuluftmenge von 1700 m³/h.

Ein weiterer Aspekt der vorliegenden Erfindung betrifft die gemäß des vorstehenden Verfahrens erhaltenen isolierten Weinsäurekerne **3** als solche.
Die erfindungsgemäß erhältlichen isolierten Weinsäurekerne **3** weisen eine gleichmäßige, kugelähnliche Geometrie auf, die die weitere Verarbeitung deutlich erleichtert. Darüber hinaus weisen die erfindungsgemäßen Pellets **3** nur geringfügige Fehlstellen durch so genannte Satelliten auf. Bei so genannten Satelliten handelt es sich um kleine, außen auf den ansonsten rundlichen Pellets anhaftenden Partikel, die die ansonsten kugelähnliche Geometrie der Pellets nachteilig beeinflussen. Von besonderer Bedeutung ist die möglichst ideale Kugelform und eine geringe Oberflächenrauhigkeit der Pellets **3** für säureempfindliche Wirkstoffe, bei denen Fehlstellen der Isolierung durch Satelliten oder zu rauher Oberfläche durch zu große Weinsäurepulverpartikel zu deutlich verschlechterten Lagerstabilitäten und damit Haltbarkeiten des fertigen Produkts führen kann.

Die Herstellung wirkstoffhaltiger Pellets aus den erfindungsgemäßen Pellets **3** kann gemäß im Stand der Technik beschriebener Verfahren erfolgen.
Ein weiterer Aspekt der vorliegenden Erfindung betrifft die Verwendung der erfindungsgemäßen Pellets **3** als Ausgangsmaterial zur Herstellung wirkstoffhaltiger Arzneimittelformulierungen.

Beispiele für die Herstellung von Pelletformulierungen des Dipyridamols ausgehend von Starterpellets finden sich in der EP32562 (insbesondere Beispiele 1 und 2). Beispiele für die Herstellung von Pelletformulierungen des Dipyridamols in Kombination mit Acetylsalicylsäure finden sich in der EP257344 (dort insbesondere Beispiel 2). Zur Herstellung von Dabigatran-haltigen Formulierungen wird beispielsweise auf die Offenbarung der WO 03/074056 verwiesen.

Die in den vorstehend genannten Dokumenten Verfahren zur Beschichtung von Starterpellets mit Wirkstoff können auch ausgehend von den erfindungsgemäßen Pellets **3** zur Anwendung gelangen.

Ein weiterer Aspekt der vorliegenden Erfindung betrifft daher die Verwendung der erfindungsgemäßen Pellets **3** als Ausgangsmaterial zur Herstellung wirkstoffhaltiger Arzneimittelformulierungen bei denen der Wirkstoff ausgewählt ist aus der Gruppe bestehend aus Dipyridamol, Dabigatran und Acetylsalicylsäure oder Kombinationen davon.

Ein bevorzugter Aspekt der vorliegenden Erfindung betrifft die Verwendung der erfindungsgemäßen Pellets **3** als Ausgangsmaterial zur Herstellung einer Dipyridamol-haltigen Formulierung. Ein weiterer bevorzugter Aspekt der vorliegenden Erfindung betrifft die Verwendung der erfindungsgemäßen Pellets **3** als Ausgangsmaterial zur Herstellung einer Dabigatran-haltigen Formulierung.

Die nachstehenden Beispiele dienen der weitergehenden Erläuterung der vorliegenden Erfindung.

### Bestimmung der Partikelgrößen der Weinsäure über Luftstrahlsiebung

**Meßgerät und Einstellungen:**

| | |
|---|---|
| Meßgerät: | Luftstrahlsieb, z.B. Alpine A 200 LS |
| Siebe: | Je nach Bedarf |
| Einwaage: | 10g/Sieb |
| Dauer: | 1 min/Sieb, danach je 1 min bis zur maximalen Gewichtsabnahme von 0,1g |

### Probenvorbereitung / Produktzufuhr:

Die Substanz wird in einen Mörser überführt und vorhandene Agglomerate durch intensives Mörsern zerstört. Das Sieb mit Gummidichtung und Deckel wird auf eine Waage gestellt, tariert und 10,0 g der gemörserten Substanz auf das Sieb eingewogen.
Das Sieb wird inklusive Inhalt, Gummidichtung und Deckel auf das Gerät gesetzt. Die Zeitschaltuhr wird auf 1 Minute eingestellt und das Material für diese Zeit per Luftstrahlsiebung behandelt. Anschließend wird der Rückstand ausgewogen und dokumentiert. Dieser Vorgang wird solange wiederholt, bis die Gewichtsabnahme des Rückstandes nach Luftstrahlsiebung < 0,1 g beträgt.

### Beispiel 1 - Herstellung der Starter Pellets

480kg Wasser werden auf 50°C erwärmt und unter Rühren mit 120 kg Acacia (Gummi Arabicum) in einem konventionellen Ansatzbehälter mit Klöpperboden und Rührorgan versetzt. Bei konstanter Temperatur wird solange gerührt, bis eine klare Lösung erhalten wird. Liegt eine klare Lösung vor (üblicherweise nach 1 bis 2 Stunden) werden unter Rühren 600 kg Weinsäure zugesetzt. Die Zugabe der Weinsäure erfolgt bei konstanter Temperatur und unter fortgesetztem Rühren. Nach beendeter Zugabe wird noch etwas 5 bis 6 Stunden nachgerührt.

1000 kg Weinsäure werden in einen langsam rotierenden (3 Umdrehungen pro Minute) nicht perforierten Horizontalkessel mit Sprüh- und Pulverauftragungseinheit (z.B. Driamat 2000/2.5) eingefüllt. Vor dem Sprühstart wird ein Muster der Säure gezogen, um sie einer Siebanalyse zu unterziehen. Bei der vorgelegten Säure handelt es sich um Weinsäurepartikel mit einer Partikelgröße im Bereich von 0,4-0,6 mm.
Auf die so vorgelegten Weinsäurepartikel wird die nach vorstehender Vorgehensweise erhaltene Säuregummilösung aufgesprüht. Beim Sprühen wird die Zuluftmenge auf 1000m³/h und 35°-75°C eingestellt. Der Differenzdruck beträgt 2mbar und die Umdrehungsgeschwindigkeit des Kessels 9 Umdrehungen pro Minute. Die Düsen werden sollten in einem Abstand von 350 - 450mm zum Füllgut angebracht sein.

Das Aufsprühen der Säuregummilösung erfolgt im Wechsel mit den folgenden Prozessschritten. Nach Aufsprühen von etwa 4,8 kg der Säuregummilösung auf die Weinsäurepartikel der Partikelgröße 0,4-0,6 mm und Verteilen der Lösung werden etwa 3,2 kg Weinsäurepulver auf die feuchten Weinsäurepartikel aufgepudert. Bei genanntem Weinsäurepulver handelt es sich um feine Weinsäurepartikel mit einer Partikelgröße von < 50 Mikrometern. Insgesamt werden 800 kg Weinsäurepulver benötigt. Nach Aufpudern mit genanntem Weinsäurepulver und weiterem Verteilen wird das Sprühgut getrocknet, bis eine Guttemperatur von etwa 40°C erreicht ist. Hieran schließt sich wieder das Aufsprühen der Säuregummilösung an.

Diese Zyklen werden so oft durchlaufen, bis die Säuregummilösung aufgebraucht ist. Nach beendetem Prozess werden die Säurepellets im Kessel bei 3 U/Min 240 Minuten lang nachgetrocknet. Um eine Verklumpung nach der Nachtrocknung zu vermeiden, erfolgt stündlich eine Intervallschaltung für 3 Minuten bei 3 U/Min. Im vorliegenden Fall bedeutet dies, dass der Kessel im Intervall von 1 Stunde 3 Minuten mit 3 U/min dreht um anschließend zu ruhen. Danach werden die Säurepellets in eine Trocknungsanlage umgefüllt. Es folgt die anschließende Trocknung bei 60°C über einen Zeitraum von 48 Stunden. Zum Schluss wird die Kornverteilung über eine Siebanalyse bestimmt. Die Korngröße mit dem Durchmesser 0,6 - 0,8mm entspricht der Gutware. Dieser Anteil sollte >85% sein.

### Beispiel 2 - Isolierung der Starter Pellets

Zur Herstellung der Isoliersuspension werden 666,1 (347,5) kg Ethanol im Ansatzbehälter vorgelegt und die Hydroxypropylmethylcellulose 33,1 (17,3) kg unter Rühren mit ca. 600 U/Min zugegeben und gelöst. Danach wird unter den gleichen Bedingungen 0,6 (0,3) kg Dimeticon zugegeben. Kurz vor Gebrauch wird Talkum 33,1 (17,3) kg, ebenfalls unter Rühren, zugegeben und suspendiert.

Die Säurepellets 1200 (600) kg werden in die Coatinganlage (z.B. GS- Coater Mod. 600/Mod. 1200) eingefüllt und dort im rotierenden Kessel in einem mehrstündigen kontinuierlichen Sprühprozess mit einer Sprührate von 32 kg/h im 1200 kg Ansatz bzw. 21 kg/h im 600 kg Ansatz mit der oben beschriebenen Isoliersuspension besprüht. Dabei werden die Pellets kontinuierlich mit einer Zuluft von bis zu 70°C getrocknet.

Nach der Entleerung des GS-Coaters werden die isolierten Starter Pellets durch Sieben fraktioniert. Die Gutfraktion mit einem Durchmesser ≤1,0 mm wird gelagert und weiter verwendet.

## Patentansprüche

1. Verfahren zur Herstellung isolierter Weinsäurepellets **3**, **dadurch gekennzeichnet, dass** in einem ersten Schritt Weinsäurepellets **1** hergestellt werden, auf die in einem zweiten Schritt eine ethanolische Isoliersuspension **2** aufgesprüht wird, die Hydroxypropylmethylcellulose enthält, wobei zur Herstellung der Weinsäurepellets **1** Weinsäurepartikel, von denen wenigstens 90 % eine Partikelgröße im Bereich von 0,4-0,6 mm, bestimmt durch Luftstrahlsiebung, aufweisen, im Wechsel mit einer Lösung aus Weinsäure und Bindemittel, bevorzugt mit Gummi Arabicum, besprüht und anschließend mit feinem Weinsäurepulver bepudert werden, wobei wenigstens 85% des Weinsäurepulvers eine Partikelgröße von <75 Mikrometern, bestimmt durch Luftstrahlsiebung, aufweisen.

2. Verfahren zur Herstellung von isolierten Weinsäurepellets **3** nach Anspruch 1 , **dadurch gekennzeichnet, dass** die verwendete ethanolische Isoliersuspension **2** neben Hydroxypropylmethylcellulose ferner Talkum enthält.

3. Verfahren zur Herstellung von isolierten Weinsäurepellets **3** nach einem der Ansprüche 1 und 2, **dadurch gekennzeichnet, dass** die verwendete ethanolische Isoliersuspension **2** neben Hydroxypropylmethylcellulose und Talkum ferner Dimethylpolysiloxan enthält.

4. Isolierte Weinsäurepellets **3** erhältlich nach einem der Ansprüche 1 bis 3.

5. Verwendung isolierter Weinsäurepellets 3 nach Anspruch 4 als Ausgangsmaterial zur Herstellung wirkstoffhaltiger Arzneimittelformulierungen.

6. Verwendung nach Anspruch5, wobei es sich um wirkstoffhaltige Arzneimittelformulierungen handelt, bei denen der Wirkstoff ausgewählt ist aus der Gruppe bestehend aus Dipyridamol, Dabigatran und Acetylsalicylsäure oder Kombinationen davon.

## Claims

1. Method of preparing insulated tartaric acid pellets **3**, **characterised in that** in a first step tartaric acid pellets **1** are prepared, onto which, in a second step, an ethanolic insulating suspension **2** containing hydroxypropylmethylcellulose is sprayed, wherein in order to prepare the tartaric acid pellets **1**, tartaric acid particles, of which at least 90% have a particle size in the range of from 0.4-0.6 mm, determined by air jet screening, are alternately sprayed with a solution of tartaric acid and binder, preferably with gum arabic, and then sprinkled with fine tartaric acid powder, wherein at least 85% of the tartaric acid powder has a particle size of < 75 microns, determined by air jet screening.

2. Method of preparing insulated tartaric acid pellets **3** according to claim 1, **characterised in that** the ethanolic insulating suspension **2** used contains talc in addition to hydroxypropylmethylcellulose.

3. Method of preparing insulated tartaric acid pellets **3** according to either claim 1 or claim 2, **characterised in that** the ethanolic insulating suspension **2** used contains dimethylpolysiloxane in addition to hydroxypropylmethylcellulose and talc.

4. Isolated tartaric acid pellets **3** which may be obtained according to one of claims 1 to 3.

5. Use of insulated tartaric acid pellets 3 according to claim 4 as starting material for the manufacture of active substance-containing medicament formulations.

6. Use according to claim 5, wherein the active substance-containing medicament formulations are those in which the active substance is selected from among dipyridamole, dabigatran and acetylsalicylic acid or combinations thereof.

## Revendications

1. Procédé de fabrication de granules d'acide tartrique isolés **(3)**, **caractérisé en ce que** sont fabriqués, lors d'une première étape, des granules d'acide tartrique **(1)**, sur lesquels est pulvérisée, lors d'une deuxième étape, une suspension d'isolation **(2)** à base d'éthanol, laquelle contient de l'hydroxypropylméthylcellulose, dans lequel, pour la fabrication des granules d'acide tartrique **(1)**, des particules d'acide tartrique, dont au moins 90 % présentent une granulométrie située dans la plage de 0,4 - 0,6 mm, définie par tamisage de jet d'air, sont aspergées en alternance d'une solution composée d'acide tartrique et d'un liant, de préférence de gomme arabique, puis sont aspergées d'une fine poudre d'acide tartrique, dans lequel au moins 85 % de la poudre d'acide tartrique présente une granulométrie < 75 micromètres, définie par- un tamisage de jet d'air.

2. Procédé de fabrication de granules d'acide tartrique isolés **(3)** selon la revendication 1, **caractérisé en ce que** la suspension d'isolation **(2)** à base d'éthanol utilisée contient en outre, en plus de l'hydroxypropylméthylcellulose, du talc.

3. Procédé servant à la fabrication de granules d'acide tartrique isolés **(3)** selon l'une quelconque des revendications 1 et 2, **caractérisé en ce que** la suspension d'isolation **(2)** à base d'éthanol utilisée contient en outre, en plus de l'hydroxypropylméthylcellulose et du talc, du diméthylpolysiloxane.

4. Granules d'acide tartrique isolés **(3)** susceptible d'être obtenus selon l'une quelconque des revendications 1 à 3.

5. Utilisation de granules d'acide tartrique isolés **(3)** selon la revendication 4 en tant que matériau de départ pour la fabrication de formulations de médicament contenant un principe actif.

6. Utilisation selon la revendication 5, dans laquelle il s'agit de formulations de médicament contenant un principe actif, dans lesquelles le principe actif est choisi parmi le groupe constitué de dipyridamole, de dabigatran, et d'acide acétylsalicylique ou de combinaisons de ces derniers.
